(19) Europäisches Patentamt
European Patent Office
Office européen des brevets

(11) **EP 2 547 381 B1**

(12) **EUROPÄISCHE PATENTSCHRIFT**

(45) Veröffentlichungstag und Bekanntmachung des Hinweises auf die Patenterteilung:
**07.11.2018 Patentblatt 2018/45**

(51) Int Cl.:
*A61M 1/36* (2006.01)   *A61M 1/10* (2006.01)
*F04D 15/00* (2006.01)

(21) Anmeldenummer: **11710128.7**

(22) Anmeldetag: **16.03.2011**

(86) Internationale Anmeldenummer:
**PCT/EP2011/001300**

(87) Internationale Veröffentlichungsnummer:
**WO 2011/113588 (22.09.2011 Gazette 2011/38)**

(54) **VORRICHTUNG ZUR DRUCK- ODER VOLUMENSTROMBESTIMMUNG VON MEDIZINISCHEN FLUIDEN**

DEVICE FOR DETERMINING THE PRESSURE OR VOLUMETRIC FLOW OF MEDICAL FLUIDS

DISPOSITIF POUR DÉTERMINER LA PRESSION OU LE DÉBIT VOLUMIQUE DE FLUIDES MÉDICAUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2010 DE 102010011798**

(43) Veröffentlichungstag der Anmeldung:
**23.01.2013 Patentblatt 2013/04**

(73) Patentinhaber: **Fresenius Medical Care Deutschland GmbH**
**61352 Bad Homburg (DE)**

(72) Erfinder:
• **HEIDE, Alexander**
**65817 Eppstein (DE)**
• **PARTENFELDER, Robin**
**61440 Oberursel (DE)**

• **PETERS, Arne**
**61352 Bad Homburg (DE)**
• **WIKTOR, Christoph**
**63571 Gelnhausen (DE)**
• **KLEWINGHAUS, Juergen**
**61440 Oberursel (DE)**

(74) Vertreter: **Bobbert & Partner**
**Patentanwälte PartmbB**
**Postfach 1252**
**85422 Erding (DE)**

(56) Entgegenhaltungen:
WO-A1-2005/085772    WO-A1-2010/149408
DE-A1- 19 645 129    DE-A1- 19 840 399
US-A- 4 211 597    US-A1- 2004 219 059
US-B1- 6 564 627

EP 2 547 381 B1

**Beschreibung**

Die vorliegende Erfindung betrifft eine Behandlungsvorrichtung gemäß Anspruch 1.

Der Druck, der in einem Volumenstrom eines Fluids oder in einer Fluidleitung herrscht, wird in der Praxis auf unterschiedliche Art gemessen.

Aus der DE 198 40 399 A1 ist eine Zentrifugalpumpenanordnung bekannt.

Aus der US 6,654,627 B1 ist ein Verfahren zum Bestimmen von Parametern einer Zentrifugalpumpenanordnung bekannt.

Aus der WO 2005/085772 A1 sind ein Verfahren und einen Anordnung zum indirekten Messen des Flusses in einer Pumpe bekannt.

Eine Aufgabe der vorliegenden Erfindung ist es, eine Behandlungsvorrichtung mit einer Recheneinheit zur Bestimmung bzw. zum Bestimmen eines Drucks eines medizinischen Fluids in einem Volumenstrom des Fluids oder in einer Fluidleitung unter Einsatz wenigstens einer Zentrifugalpumpe anzugeben.

Diese erfindungsgemäße Aufgabe wird durch eine Behandlungsvorrichtung mit den Merkmalen des Anspruchs 1 gelöst.

Das offenbarte, nicht-erfindungsgemäße Verfahren umfasst das Ermitteln des Drucks stromauf der Zentrifugalpumpe oder einer Impellerpumpe unter Berücksichtigung einer Angabe über einen Druck stromab der Zentrifugalpumpe oder das Ermitteln des Drucks stromab der Zentrifugalpumpe oder einer Impellerpumpe unter Berücksichtigung einer Angabe über einen Druck stromauf der Zentrifugalpumpe. In jedem dieser Fälle umfasst das Verfahren dabei ein Berücksichtigen einer Angabe über den Volumenstrom in der Fluidleitung und einer Angabe wenigstens einer Drehzahl der Zentrifugalpumpe.

Ein weiteres offenbartes, nicht-erfindungsgemäßes Verfahren umfasst das Ermitteln des Volumenstroms in der Fluidleitung, oder einer Angabe hierüber, unter Berücksichtigen des Drucks stromauf und des Drucks stromab der Zentrifugalpumpe sowie wenigstens einer Angabe über eine Drehzahl der Zentrifugalpumpe.

Vorteilhafte Weiterbildungen der erfindungsgemäßen Behandlungsvorrichtung sind jeweils Gegenstand der Unteransprüche und Ausführungsformen.

Erfindungsgemäße Ausführungsformen können jeweils eines oder mehrere der im Folgenden genannten Merkmale aufweisen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Unter dem Begriff "Druck", wie er hierin verwendet wird, ist in einer erfindungsgemäßen Ausführungsform der statische Druck des Fluids (auch als hydrostatischer Druck bezeichnet) im Volumenstrom oder in der Fluidleitung zu verstehen.

In einer anderen erfindungsgemäßen Ausführungsform ist unter "Druck" der dynamische Anteil (auch als hydrodynamischer Druck bezeichnet) des Fluids zu verstehen.

Der statische Druck kann mittels geeigneter, aus dem Stand der Technik bekannter Drucksensoren oder Druckaufnehmer gemessen werden. Beispiele für derartige Drucksensoren sind piezoelektrische Drucksensoren oder Drucksensoren mit Dehnungsmessstreifen.

Unter einem Bestimmen eines Drucks wird in manchen erfindungsgemäßen Ausführungsformen ein Berechnen des Drucks oder ein Abschätzen des Drucks basierend auf mathematischen Formeln in ausreichend guter Annäherung oder dergleichen verstanden.

Unter dem Begriff "medizinisches Fluid", wie er hierin verwendet wird, wird Blut, Dialysat, Substituat oder jedes andere medizinische Fluid verstanden. Dazu gehören beispielsweise auch Fluide, um einen Schlauch oder Kreislauf vor oder nach einer Anwendung zu reinigen, vor einer Anwendung zu füllen ("Priming Volume"). Ferner zählen zu den medizinischen Fluiden Dialysierflüssigkeiten, gelöste Substanzen wie Elektrolyte (Natrium, Kalium, Calcium, Magnesium), Puffer (Lactat oder Bicarbonat), Zucker (Glucose) und dergleichen. Ein medizinisches Fluid kann auch eine geeignete Kombination von manchen der vorstehend genannten Fluide sein.

Zentrifugalpumpen im Sinne der vorliegenden Erfindung können beispielsweise als Radialpumpen, Diagonal- oder Halbaxialpumpen oder Axialpumpen ausgeführt sein.

Die Begriffe "stromauf" bzw. "stromaufwärts" und "stromab" bzw. "stromabwärts" der Zentrifugalpumpe beziehen sich auf die Durchströmungsrichtung der Zentrifugalpumpe oder auf die Ein- bzw. Ausströmrichtung des medizinischen Fluids bezogen auf die Zentrifugalpumpe.

"Stromauf" bezieht sich auf Orte und Zustände vor der Durchströmung der Zentrifugalpumpe, also vor dem Pumpeneintritt. Dies kann ein Ort innerhalb der Einstromleitung unmittelbar vor dem Pumpeneintritt sein, z. B. 1 cm, 5 cm, 10 cm vor der Zentrifugalpumpe, oder in weiterem Abstand, z. B. 50 cm oder 100 cm, oder in zwischen diesen Werten/Abständen liegenden Abständen, vor der Zentrifugalpumpe.

"Stromab" bezieht sich auf Orte und Zustände nach der Durchströmung der Zentrifugalpumpe, also auf einen Ort innerhalb der Ausstromleitung nach dem Pumpenaustritt. Über die Abstände gilt das Gleiche wie zu "stromauf". Der Druck stromab wird regelmäßig auch als Postpumpendruck bezeichnet.

Eine Angabe über den Volumenstrom im Sinne der vorliegenden Erfindung kann auf unterschiedliche Weise ermittelt werden. Beispielsweise kann eine Angabe mittels geeigneter Volumenstromsensoren (auch als Durchflusssensoren oder Durchfluss-Messaufnehmer bezeichnet) erhalten bzw. gemessen werden.

Volumenstromsensoren basieren beispielsweise auf magnetischinduktiven Messprinzipien oder auf Ultraschall-Messverfahren.

Der Volumenstrom kann auch indirekt mittels einer Drosselvorrichtung und Druckmessungen vor und hinter der Drosselvorrichtung bestimmt werden.

Die Volumenstrommessung, die vorgeschrieben sein kann, kann bei Dialysemaschinen mit Waagen für Dialysat- oder Substituatlösungsbeuteln durch die Auswertung von Waagensignalen geschehen. Eine solche Ausführung ist beispielsweise im Stand der Technik bekannt. Hierbei kann vorteilhaft auf einen Flusssensor (bzw. Volumenstromsensor) verzichtet werden.

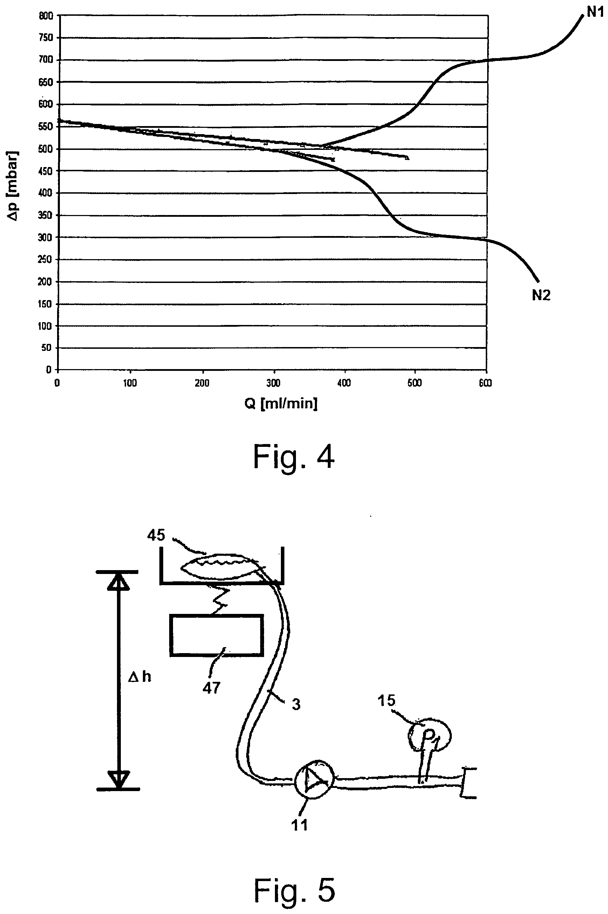

Eine solche Messung kann insbesondere bei Dialysat- oder Substituatlösungen, deren Dichte bekannt ist und somit aus dem (ggf. transienten) Waagensignal ein Volumen-pro-Zeitsignal (Fluss) abgeleitet werden kann, verwendet werden. Eine geeignete Anordnung ist in Fig. 5 veranschaulicht.

Die Drehzahl der Zentrifugalpumpe bestimmt sich in einer Ausführungsform des Verfahrens anhand der elektrischen Eingangsleistung der Zentrifugalpumpe. Die Eingangsleistung kann dabei auch von weiteren Parametern, wie z. B. der Förderrate und/oder der Viskosität des geförderten Fluids abhängen. Solche Parameter können ebenfalls berücksichtigt sein. Entsprechend vorbereitete Einrichtungen können vorgesehen sein.

Die Drehzahl kann zu der elektrischen Eingangsspannung der Zentrifugalpumpe proportional sein, die Förderrate und/oder die Viskosität kann zum Eingangsstrom proportional sein. Die elektrische Eingangsleistung ist zur Eingangsspannung und zum Eingangsstrom proportional.

In anderen erfindungsgemäßen Ausführungsformen wird die Drehzahl über Hall-Sensoren, die das magnetische Feld eines (Permanent-)Magneten der Zentrifugalpumpe detektieren, erfasst.

Drehzahlmessungen können auch mittels weiterer, dem Fachmann bekannter Verfahren bestimmt werden, wie beispielsweise optischen Verfahren (z. B. stroboskopisch), Verfahren basierend auf dem Induktionsgesetz (Tachogenerator, Induktionsgeber etc.) usw.

In anderen erfindungsgemäßen Ausführungsformen ist die Drehzahl der Pumpe direkt vom Betriebssystem vorgegeben. Die Drehzahl der Pumpe kann somit bekannt sein.

Erfindungsgemäß kann der Druck oder eine Angabe hierüber stromauf der Zentrifugalpumpe bestimmt werden, wenn Parameter wie der Druck stromabwärts der Zentrifugalpumpe, der Volumenstrom und die Drehzahl der Zentrifugalpumpe bei Betrieb bekannt sind. Dabei genügt es in manchen Ausführungsformen der Erfindung, wenn anstelle des Volumenstroms die Eingangsleistung der Zentrifugalpumpe sowie ggf. ein zusätzlicher Wert bekannt sind, welche einen ausreichend eindeutigen Rückschluss auf den Volumenstrom erlauben.

Ebenso genügt es in anderen Ausführungsformen der Erfindung, wenn anstelle der Drehzahl der Zentrifugalpumpe andere Werte vorliegen oder gemessen oder ermittelt wurden, welche einen ausreichend eindeutigen Rückschluss auf die Drehzahl erlauben.

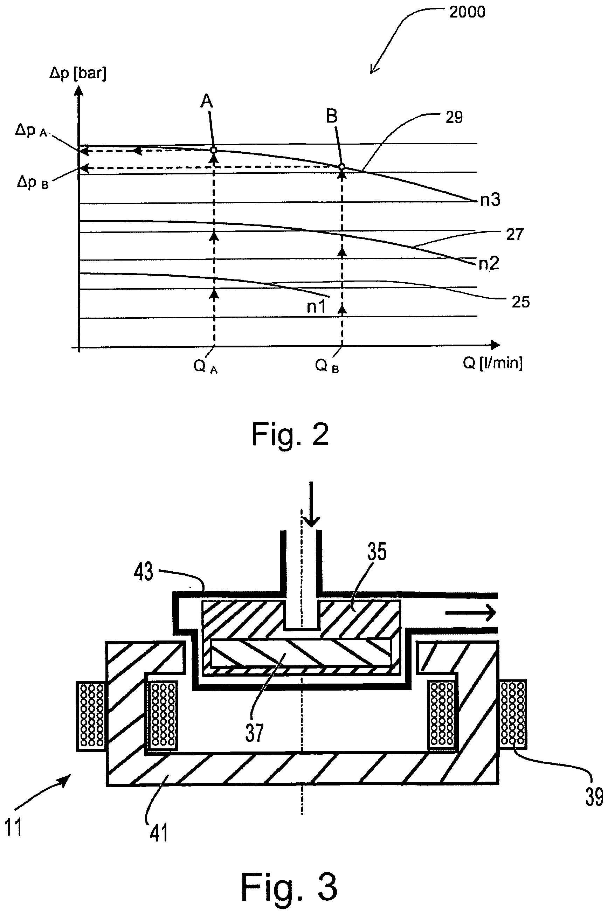

Das Ermitteln eines Werts für den Druck stromauf der Zentrifugalpumpe kann beispielsweise rechnerisch anhand von Werten erfolgen, welche aus einem Kennlinienfeld (wie es beispielsweise in Fig. 2 gezeigt ist) der verwendeten Zentrifugalpumpe zu entnehmen sind. Dabei wird vor allem auf den üblicherweise von der Ordinate bzw. y-Achse abgelesenen Wert der Druckdifferenz Bezug genommen. Durch Kenntnis der Druckdifferenz und Kenntnis eines Druckwerts stromauf der Zentrifugalpumpe kann der Druck stromab berechnet werden, und umgekehrt. Die Kenntnis des Volumenstroms gibt dabei Auskunft über die genaue Lage der Kennlinie im Diagramm und damit Auskunft über die Druckdifferenz.

Für Drehzahlen oder Drehzahldifferenzen, die nicht als Kennlinien im jeweils verwendeten Kennlinienfeld aufgetragen sind, kann durch Interpolation ein angenäherter Wert ermittelt werden.

Erfindungsgemäß kann der Druck oder eine Angabe hierüber stromauf der Zentrifugalpumpe bereits vorliegen oder bekannt sein, wenn ein hydrostatischer Druck oder die Höhe einer Flüssigkeitssäule bis zum Pumpeneingang bekannt ist. Bei derartigen Ausführungsformen kann ein ansonsten stromaufwärts vorgesehener Drucksensor vorteilhaft entfallen.

In einer erfindungsgemäßen Ausführungsform wird der Druck des medizinischen Fluids in einem extrakorporalen Volumenstrom bestimmt.

Der Begriff "extrakorporaler Volumenstrom", wie er hierin verwendet wird, bezeichnet einen Volumenstrom außerhalb eines Körpers eines Patienten. Dabei kann es sich um einen menschlichen oder tierischen Patienten handeln. Es spielt erfindungsgemäß keine Rolle, ob der Patient krank oder gesund ist.

Der extrakorporale Volumenstrom kann ein geschlossener oder offener Kreislauf sein.

Der extrakorporale Volumenstrom kann einen arteriellen und/oder einen venösen Zugang zum Körper haben.

In einer weiteren Ausführungsform wird der Druck unter Berücksichtigung einer Angabe über den Volumenstrom am Ein- oder am Ausgang der Zentrifugalpumpe bestimmt.

Der Druck stromauf oder stromab der Zentrifugalpumpe kann jeweils ein Über- oder Unterdruck sein.

In bestimmten erfindungsgemäßen Ausführungsformen ist die Viskosität des geförderten Fluids bekannt. Die Viskosität kann die Pumpenkennlinie beeinflussen. Ihre Kenntnis und deren Berücksichtigung kann vorteilhaft die Genauigkeit erhöhen. Eine Pumpenkennlinie ist beispielsweise in Fig. 4 gezeigt.

In manchen erfindungsgemäßen Ausführungsformen erfolgt die Viskositätsmessung durch die Pumpe selbst, wie dies beispielsweise im europäischen Patent EP 1 284 415 B1 beschrieben ist.

In bestimmten erfindungsgemäßen Ausführungsformen erfolgt die relative oder absolute Viskositätsmessung durch andere Mittel, wie zum Beispiel mit Hilfe optischer Hämatokritmessungen.

In manchen anderen Ausführungsformen der vorliegenden Erfindung wird der Wert der Viskosität dem System durch Eingabe bekannt gemacht.

Die dem Verfahren zugrunde liegenden Prinzipien basieren im Wesentlichen darauf, dass anhand eines Kennlinienfeldes der verwendeten (Zentrifugal-)Pumpe und eines bekannten Volumenstroms eine zugehörige Druckdifferenz abgelesen werden kann. Die abgelesene Druckdifferenz lässt nach Messen eines Druckwerts des Fluids in der Fluidleitung vor (stromauf) oder nach (stromab) der Zentrifugalpumpe Rückschlüsse bzw. eine Berechnung des anderen Druckwerts des Fluids in der Fluidleitung nach (stromab) oder vor (stromauf) der Zentrifugalpumpe zu.

Alternativ dazu können die dem Verfahren zugrunde liegenden Prinzipien dazu eingesetzt werden, den Volumenstrom bzw. Fluss eines medizinischen Fluids in einer Fluidleitung zu bestimmen, unter Einsatz wenigstens einer Zentrifugalpumpe. Hierbei werden die Druckwerte des Fluids stromauf und stromab der Zentrifugalpumpe ermittelt. Zwischen diesen Druckwerten wird die Druckdifferenz berechnet. Der zugehörige Volumenstrom bzw. Fluss des Fluids wird anschließend anhand des Kennlinienfeldes und der zugehörigen Drehzahl der Pumpe ermittelt. Auch dieses Verfahren, welches dann ein Verfahren zum Bestimmen eines Volumenflusses ist, ist Gegenstand der vorliegenden Offenbarung. Ausführungsformen hiervon weisen eines oder mehrere der hierin an beliebiger Stelle genannten Merkmale und Merkmalskombinationen auf, sofern dies für den Fachmann erkennbar sinnvoll ist.

Die mit dem offenbarten Verfahren erzielbaren Vorteile sind ungeschmälert auch mit der offenbarten, nichterfindungsgemäßen externen medizinischen Funktionseinrichtung erzielbar.

In einer Ausgestaltung ist die externe medizinische Funktionseinrichtung derart ausgestaltet, dass sie zur Bestimmung eines Drucks eines Fluids stromauf einer Zentrifugalpumpe nur einen Drucksensor oder eine Druckmessstelle stromab der Zentrifugalpumpe oder zur Bestimmung eines Drucks stromab der Zentrifugalpumpe nur einen Drucksensor oder eine Druckmessstelle stromauf der Zentrifugalpumpe aufweist. Zusätzliche Drucksensoren oder Druckmessstellen zur Messung eines Drucks eines anderen Fluids oder desselben Fluids an anderer Stelle oder in einem anderen Zustand bleiben hiervon unberührt. So kann die Zentrifugalpumpe im arteriellen Schenkel eines Blutkreislaufs angeordnet sein, um mittels des Verfahrens den arteriellen Druck stromauf der Zentrifugalpumpe zu bestimmen. Zusätzlich kann das System, welches die Zentrifugalpumpe aufweist, an anderer Stelle (z. B. in einem venösen Schenkel des Blutkreislaufs) weitere Druckmesser, z. B. venöse Druckmesser, aufweisen. Dies trifft auch auf die erfindungsgemäße Behandlungsvorrichtung in einigen erfindungsgemäßen Ausführungsformen hiervon zu.

In einer Ausgestaltung weist die externe medizinische Funktionseinrichtung wenigstens einen sich drehenden bzw. rotierenden Abschnitt bzw. einen Rotationsabschnitt auf.

Dieser ist in einer Ausgestaltung mechanisch gelagert, in einer anderen ist er magnetisch gelagert.

Der Rotationsabschnitt kann ausschließlich oder ergänzend magnetisch gelagert sein.

Der Rotationsabschnitt kann in einem Inneren der medizinischen Funktionseinrichtung angeordnet sein.

Der Rotationsabschnitt ist in einer Ausführungsform ein Laufrad oder ein Rotor.

In einer weiteren Ausgestaltung weist die externe medizinische Funktionseinrichtung wenigstens einen Rotationsabschnitt auf, welcher vorgesehen und ausgestaltet ist, um mittels eines externen Antriebs magnetisch oder mittels eines elektrischen Feldes antreibbar zu sein oder betrieben zu werden.

In einer weiteren Ausführungsform ist der externe Antrieb des Rotationsabschnitts ausgestaltet, um mechanisch, z. B. über lösbare, fluiddichte Kupplungen angetrieben zu werden.

Die magnetische Antriebskraft oder -wirkung kann mit Hilfe eines oder mehrerer Magneten erzielt werden. Sie kann mit Hilfe von stromdurchflossenen Leitern erzielt werden. Beispielsweise können stromdurchflossene Spulen verwendet werden.

Eine solche magnetisch angetriebene Zentrifugalpumpe kann den Vorteil bieten, dass keine mechanische und/oder elektrische Schnittstelle zur antreibenden Vorrichtung oder Maschine erforderlich ist und/oder keine Fluide von der Vorrichtung, wie beispielsweise einer (medizinischen) Behandlungsvorrichtung, zur Zentrifugalpumpe übertragen werden müssen.

In einer Ausgestaltung ist die externe medizinische Funktionseinrichtung als externer Flüssigkeitskreislauf mit Substituat-, Dialysat- und/oder extrakorporalem Blutkreislauf ausgestaltet.

In einer Ausgestaltung ist die externe medizinische Funktionseinrichtung als Blut- bzw. Dialysatkassette oder kombinierte Blut-/Dialysatkassette ausgestaltet.

In einer Ausgestaltung ist die externe medizinische Funktionseinrichtung ein Disposable, ein Einmalartikel oder ein Einweg-Produkt.

In manchen Ausgestaltungen ist die externe Funktionseinrichtung eine Funktionseinrichtung, die nicht dauerhafter Bestandteil einer Behandlungsvorrichtung ist sondern vielmehr dieser "von außen kommend" an- oder beigefügt bzw. mit dieser zum Zweck der Behandlung verbunden wird.

In einer Ausgestaltung ist die externe medizinische Funktionseinrichtung eine Disposable-Kassette.

Die Disposable-Kassette kann ein Hartteil sein. Sie kann aus einem Kunststoffmaterial hergestellt sein. Die Disposable-Kassette kann mittels eines Spritzgussverfahrens hergestellt sein.

Ausführungen zur weiteren Ausgestaltung der externen medizinischen Funktionseinrichtung sind in der von der Anmelderin der vorliegenden Erfindung beim DPMA am 16. Dezember 2009 eingereichten Anmeldung 10 2009 058 681.4 mit dem Titel "Bilanziereinrichtung, externe medizinische Funktionseinrichtung, Behandlungsvorrichtung sowie Verfahren" offenbart.

In bestimmten Ausgestaltungen weist die externe medizinische Funktionseinrichtung wenigstens einen oder genau einen Drucksensor und/oder wenigstens eine oder genau eine Drucksensor-Koppelstelle auf, insbesondere vorgesehen zur Durchführung des Verfahrens.

In manchen Ausgestaltungen weist die externe medizinische Funktionseinrichtung wenigstens einen oder genau einen Volumensensor und/oder wenigstens eine oder genau eine Volumensensor-Koppelstelle auf, insbesondere vorgesehen zur Durchführung des Verfahrens.

In einigen Ausgestaltungen ist die externe medizinische Funktionseinrichtung eine Blutkassette. In manchen Ausgestaltungen ist die externe medizinische Funktionseinrichtung eine Dialyse-, insbesondere eine Hämodialyse-Blutkassette.

Die Recheneinrichtung kann ein Computer oder ein Mikroprozessor sein, der beispielsweise Messsignale des Drucks stromauf oder stromab der Zentrifugalpumpe, des Volumenstroms und der Drehzahl der Zentrifugalpumpe erfassen und weiterverarbeiten kann und hierzu konfiguriert ist.

Die Recheneinrichtung kann auch dazu verwendet werden, um die Messsignale zu verarbeiten und daraus errechnete Daten als Steuer- und/ oder Regelsignale an die Zentrifugalpumpe weiterzuleiten. Dies kann beispielsweise dann vorteilhaft sein, wenn der Volumenstrom und/oder der gemessene und/oder der erfindungsgemäß bestimmte Druck bestimmte Grenz- oder Schwellwerte unter- oder überschreitet.

Die mit dem Verfahren erzielbaren Vorteile sind ungeschmälert auch mit der erfindungsgemäßen Behandlungsvorrichtung erzielbar.

Die erfindungsgemäße Behandlungsvorrichtung ist ausgestaltet und konfiguriert zum Bestimmen eines Drucks gemäß dem offenbarten Verfahren. Hierzu weist die Behandlungsvorrichtung in einer erfindungsgemäßen Ausführungsform eine Recheneinrichtung auf, die geeignet und vorgesehen bzw. konfiguriert ist zum Bestimmen eines Drucks nach dem Verfahren.

In einer erfindungsgemäßen Ausführungsform ist die Behandlungsvorrichtung derart ausgestaltet, dass sie zur Bestimmung eines Drucks stromauf einer Zentrifugalpumpe nur einen Drucksensor stromab, oder zur Bestimmung eines Drucks stromab der Zentrifugalpumpe nur einen Drucksensor stromauf der Zentrifugalpumpe aufweist.

In einer erfindungsgemäßen Ausführungsform umfasst die Behandlungsvorrichtung wenigstens einen arteriellen Leitungsabschnitt, eine Zentrifugalpumpe zum Fördern von Blut in dem extrakorporalen Blutkreislauf und einen venösen Leitungsabschnitt.

In einer weiteren Ausführungsform ist die Behandlungsvorrichtung als Blutbehandlungsvorrichtung, insbesondere als Hämodialysevorrichtung, ausgestaltet.

In einer Ausführungsform der erfindungsgemäßen Behandlungsvorrichtung weist die Behandlungsvorrichtung eine Einrichtung auf, die dazu vorgesehen und konfiguriert ist, um die Zentrifugalpumpe über eine magnetische Antriebsschnittstelle anzutreiben.

Die Einrichtung kann beispielsweise ein Magnet oder ein magnetisch wirkendes System und/oder ein stromdurchflossener Leiter, wie beispielsweise eine oder mehrere stromdurchflossene Spulen, sein oder solche Einrichtungen aufweisen.

Eine Ausgabeeinheit kann an die Recheneinrichtung angeschlossen sein, um den erfindungsgemäß bestimmten Druck anzeigen zu können.

Selbst wenn dies nicht ausdrücklich erwähnt ist, weist die erfindungsgemäße Behandlungsvorrichtung jene Einrichtungen auf, die zur Ausführung des Verfahrens entsprechend dessen jeweiliger Ausführungsform erforderlich sind.

Das digitale Speichermedium, welches insbesondere eine Diskette, eine CD oder eine DVD ist, weist bevorzugt elektrisch auslesbare Steuersignale auf, die so mit einem programmierbaren Computersystem zusammenwirken können, dass die maschinellen Schritte des Verfahrens veranlasst werden.

Das Computer-Programm-Produkt weist vorzugsweise einen auf einem maschinenlesbaren Träger gespeicherten Programmcode zur Veranlassung der maschinell durchführbaren Schritte des Verfahrens, wenn das Programm-Produkt auf einem Rechner abläuft, auf.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD und dergleichen sein.

Das Computer-Programm weist einen Programmcode zur Veranlassung der maschinell durchführbaren Schritte des Verfahrens auf, wenn das Programm auf einem Rechner oder Computer abläuft.

Mittels der vorliegenden Erfindung ist es vorteilhaft möglich, zu praktisch einem Zeitpunkt zwei Drücke unter Einsatz nur eines Drucksensors oder allgemein nur einer Druckmessstelle zu bestimmen. Daher kann erfindungsgemäß gegenüber dem Stand der Technik vorteilhaft mit nur einem Drucksensor ein zusätzlicher zweiter Druck bestimmt werden. Alternativ kann zur Bestimmung von zwei Drücken (stromauf und stromab) auf das Vorsehen eines Drucksensors verzichtet werden. Letzteres trägt zur Senkung des Herstellungsaufwands sowie der Herstellungskosten bei. Da es im Zusammenhang mit dem zweiten, erfindungsgemäß nicht erforderlichen, Drucksensor nicht zu, durch diesen bedingten, Artefakten oder Fehlanzeigen kommen kann, kann die Güte der Druckmessung vorteilhaft gesteigert werden.

In der Praxis kam es vor allem bei Druckmessern, welche im Bereich von bestimmten Unterdrücken gemessen haben (wie dies im arteriellen Schenkel eines extrakorporalen Kreislaufs regelmäßig der Fall ist), immer wieder zu technischen Unzulänglichkeiten aufgrund von Kontaktverlusten zwischen Druckaufnehmer und Druckgeber wie Membran oder dergleichen. Derartige Unzulänglichkeiten treten bei Einsatz der vorliegenden Erfindung durch Verzicht auf einen Drucksensor (welcher je nach seinem Einsatz andernfalls insbesondere Unterdrücke messen würde) vorteilhaft nicht auf.

Derartige Unzulänglichkeiten wurden im Stand der Technik oftmals mit besonderem technischem Aufwand behoben. Dieser Aufwand kann erfindungsgemäß vorteilhaft unterbleiben.

Ferner können durch Verzicht auf einen weiteren Drucksensor oder eine weitere Druckmessstelle die Abmessungen der externen medizinischen Funktionseinrichtung bzw. ihr Platzbedarf vorteilhaft gering gehalten werden.

Durch eine magnetische Lagerung der Zentrifugalpumpe kann die Konstruktion der externen medizinischen Funktionseinrichtung vorteilhaft vereinfacht werden. So kann es vorteilhaft möglich sein, auf mechanische Bauteile, wie Lager und dergleichen, zu verzichten und auf diese Weise vorteilhaft einen geringen Verschleiß der Bauteile und/oder einen geringen Partikelabrieb sicherzustellen. Hierdurch bedingt kann vorteilhaft auch eine Erwärmung der Zentrifugalpumpe vermieden oder verringert werden.

Die magnetische Antriebsschnittstelle zum Betreiben der Zentrifugalpumpe bzw. eines Rotationsabschnitts derselben kann vorteilhaft einen berührungslosen und/oder dichtungsfreien Antrieb der Zentrifugalpumpe bereitstellen. Auf diese Weise kann es vorteilhaft möglich sein, auf offene Schnittstellen zwischen der Zentrifugalpumpe und der Behandlungsvorrichtung zu verzichten.

Eine - wenngleich nur äußerst geringe - Kontaminationsgefahr der medizinischen Fluide kann somit vorteilhaft verringert oder sogar ganz ausgeschlossen werden.

Ein Durchführen des Verfahrens mittels einer externen, nichterfindungsgemäßen Funktionseinrichtung kann durch das Zusammenfassen verschiedener oder aller Funktionen und/oder der hierfür erforderlichen Bauteile auf einer Funktionseinrichtung, welche vorzugsweise eine Blutkassette, insbesondere eine Dialyse-, ganz insbesondere eine Hämodialyse-Blutkassette ist, ein kostengünstiges Herstellen der verwendeten Bauteile wie Sensoren oder Pumpenabschnitte erlauben.

Ferner kann das Zusammenfassen wie zuvor beschrieben in bestimmten erfindungsgemäßen Ausführungsformen vorteilhaft ein vereinfachtes, da gemeinsames Anordnen aller oder mancher der zum Durchführen des Verfahrens erforderlichen Bauteile an einer Behandlungsvorrichtung erlauben.

Zudem kann das Zusammenfassen wie zuvor beschrieben in manchen erfindungsgemäßen Ausführungsformen vorteilhaft ein vergleichsweise sicheres Einlegen aller oder mancher der zum Durchführen des Verfahrens erforderlichen Bauteile in oder an einer Behandlungsvorrichtung erlauben. Dies kann gewährleistet sein durch deren Anordnung in oder auf der externen Funktionseinrichtung unter bestimmten Abständen, unter bestimmen Verbindungen zueinander oder mit weiteren Bauteilen, usw.

Im Folgenden wird die vorliegende Erfindung beispielhaft unter Bezugnahme auf die beigefügte Zeichnung beschrieben. In der Zeichnung bezeichnen identische Bezugszeichen gleiche oder identische Elemente. Es gilt:

Fig. 1     zeigt schematisch vereinfacht einen extrakorporalen Blutkreislauf mit einer Zentrifugalpumpe zur Durchführung des Verfahrens;

Fig. 2     zeigt beispielhaft ein Kennlinienfeld einer Zentrifugalpumpe;

Fig. 3     zeigt schematisch vereinfacht eine Zentrifugalpumpe mit magnetischer Lagerung und magnetischem Antrieb;

Fig. 4     zeigt Kennlinien einer Zentrifugalpumpe in Abhängigkeit von der Viskosität eines Fluids; und

Fig. 5     zeigt schematisch eine Anordnung zur Volumenstrommessung.

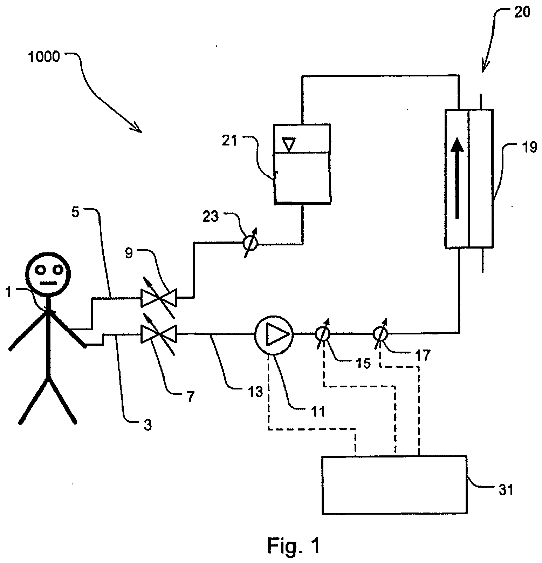

In **Fig. 1** ist schematisch vereinfacht ein extrakorporaler Blutkreislauf 1000 zur Durchführung des Verfahrens an einem Patienten 1 dargestellt.

Der extrakorporale Blutkreislauf 1000 weist einen arteriellen Schenkel bzw. arteriellen Leitungsabschnitt 3 sowie einen venösen Schenkel bzw. venösen Leitungsabschnitt 5 auf. Im arteriellen Leitungsabschnitt 3 des extrakorporalen Blutkreislaufs 1000 befindet sich ein arterielles Absperrventil 7; im venösen Leitungsabschnitt 5 des extrakorporalen Blutkreislaufs 1000 befindet sich ein venöses Absperrventil 9.

Eine Zentrifugalpumpe 11 erzeugt den notwendigen Perfusionsdruck zur Durchströmung des extrakorporalen Blutkreislaufs 1000. Dabei kann, je nach den Druckverhältnissen im arteriellen Leitungsabschnitt 3 und in einem Ansaugbereich 13, ein Unterdruck im Ansaugbereich 13 entstehen.

Ein Drucksensor 15 misst den Druck stromab der Zentrifugalpumpe 11, der im Wesentlichen von der Zentrifugalpumpe 11 erzeugt bzw. aufgebaut wird. Dieser Druck ist in dieser Anordnung zugleich der Eingangsdruck für die nachfolgende Blutbehandlungseinrichtung 19, beispielsweise einen Hämodialysator, einer nur abschnittsweise dargestellten erfindungsgemäßen Behandlungsvorrichtung 20.

Stromab des Drucksensors 15 ist im extrakorporalen Blutkreislauf 1000 ein Volumenstromsensor 17 angeordnet. Dies kann beispielsweise ein magnetisch-induktiver Sensor oder ein Ultraschall-Sensor sein.

Stromab der Blutbehandlungseinrichtung 19 ist eine venöse Tropfkammer 21 angeordnet.

Zwischen der venösen Tropfkammer 21 und dem venösen Absperrventil 9 ist ein venöser Drucksensor 23 angeordnet.

Erfindungsgemäß braucht kein arterieller Drucksensor im Ansaugbereich 13 bzw. stromauf der Zentrifugalpumpe 11 angeordnet zu sein, um den Druck stromauf der Zentrifugalpumpe 11 bestimmen zu können. Dieser Druck stromauf der Zentrifugalpumpe 11 wird erfindungsgemäß vielmehr mit Hilfe von Angaben über den Volumenstrom, den Druck stromab der Zentrifugalpumpe 11 und deren Drehzahl bestimmt.

In Fig. 1 ist ferner eine Recheneinrichtung 31 dargestellt, mittels welcher die Bestimmung des Drucks nach dem Verfahren erfolgt.

In der folgenden Diskussion der Fig. 2 wird zum besseren Verständnis auch Bezug genommen auf Einrichtungen, die in Fig. 1, nicht aber erneut in Fig. 2 dargestellt sind.

In **Fig. 2** ist beispielhaft ein Kennlinienfeld 2000 der Zentrifugalpumpe 11 dargestellt.

Das Kennlinienfeld 2000 beschreibt das Betriebsverhalten der Zentrifugalpumpe 11 und wurde zuvor experimentell ermittelt. Exemplarisch sind drei Kennlinien für jeweils eine Drehzahl n1 (Bezugszeichen 25), n2 (Bezugszeichen 27) und n3 (Bezugszeichen 29) dargestellt.

Beispielhaft werden zwei Betriebspunkte A und B der Zentrifugalpumpe 11 im Kennlinienfeld 2000 erläutert, um das erfindungsgemäße Bestimmen des Drucks stromauf der Zentrifugalpumpe 11 im Ansaugbereich 13 zu verdeutlichen.

Mithilfe einer Steuerkonsole der Zentrifugalpumpe 11, die auch als Recheneinrichtung 31 für die Aufnahme und Verarbeitung der verschiedenen Messgrößen dient, wird ein vorgegebener oder gewünschter Volumenstrom, z. B. $Q_A$, eingestellt, der mit dem Volumenstromsensor 17 auch gemessen werden kann. Ist der gewünschte Volumenstrom $Q_A$ erreicht, dreht die Zentrifugalpumpe 11 mit einer dazu korrelierenden Drehzahl, beispielsweise mit der Drehzahl n3 (Bezugszeichen 29).

Mithilfe der zur Drehzahl n3 gehörigen Kennlinie des Kennlinienfeldes 2000 kann jetzt der Druckunterschied $\Delta p_A$ an der Ordinate abgelesen werden. Der Druck am Auslass oder stromab der Zentrifugalpumpe 11 wird mit dem Drucksensor 15 gemessen. Der Druck stromauf oder am Einlass der Zentrifugalpumpe 11 im Ansaugbereich 13 kann jetzt erfindungsgemäß bestimmt bzw. errechnet werden.

Das folgende, weitere exemplarische Rechenbeispiel verdeutlicht nochmals diese Vorgehensweise:

Die Zentrifugalpumpe 11 arbeitet bei diesem Beispiel mit einer Drehzahl von 3000 U/min und es stellt sich ein Volumenstrom von ca. 300 ml/min ein. Mittels der Kennlinie wird eine Druckdifferenz $\Delta p$ von ca. 260 mbar an der Ordinate abgelesen. Weiterhin wird ein Druck stromab der Zentrifugalpumpe 11 von 200 mbar gemessen. Daraus lässt sich der Druck stromauf der Zentrifugalpumpe 11 zu -60 mbar bestimmen (Druckdifferenz $\Delta p$ = p stromab minus p stromauf; 260 mbar = 200 mbar - p stromauf).

In allen Fällen können entsprechende Regelparameter mit Störgrößen-Betrachtungen in die Regelung bzw. Steuerung des Durchflusses durch den extrakorporalen Blutkreislauf 1000 und/oder in die Antriebssteuerung der Zentrifugalpumpe 11 integriert werden.

**Fig. 3** zeigt eine Zentrifugalpumpe 11 mit einem Rotor 35 als Rotationsabschnitt, einem in den Rotor 35 eingelassenen Permanentmagneten 37, Spulen 39 und einem Stator 41. Die Zentrifugalpumpe 11 weist ein Gehäuse 43 auf mit einem Zulass und einem Auslass (durch Pfeile in Fig. 3 als solche erkennbar).

Die Zentrifugalpumpe 11 wird in der gezeigten Strömungsrichtung durchströmt. Der Antrieb des Rotors 35 erfolgt durch ein umlaufendes elektromagnetisches Feld, erzeugt mittels Ansteuern der Spulen 39 des Stators 41.

Im bzw. am Rotor 35 können ein oder mehrere Laufradmagnete oder wenigstens ferromagnetische Materialien integriert sein.

Die Lagerung des Rotors 35 kann dann einerseits mittels der Laufradmagnete und andererseits mittels außerhalb der Zentrifugalpumpe 11 vorgesehener Magnete erfolgen. Die Magnete können umlaufend in der gleichen Drehbewegung wie der Rotor 35 angeordnet sein. Anstelle der umlaufenden Magnete oder ergänzend hierzu kann auch ein umlaufendes elektromagnetisches Feld in einer Spulenanordnung den Rotor 35 lagern bzw. in einer stabilen umlaufenden Position fixieren. Diese Ausführungsform ist - obgleich nicht in den Figuren dargestellt - ebenfalls von der Erfindung umfasst.

**Fig. 4** zeigt Kennlinien N1 und N2 (dabei gilt: $\Delta P = f(V)$) der Zentrifugalpumpe 11 in Abhängigkeit von der dynamischen Viskosität eines Fluids. Die Einheit der Viskosität ist in Centipoise (cP; 1 cP = $10^{-3}$ kg/ms, alternativ mPa*s) angegeben.

Die Messwerte der Kennlinie N1 wurden bei einer Viskosität von 3 cP erfasst. Die die Messwerte verbindende Kennlinie N1 wurde polynomisch an die Messwerte angenähert.

Die Messwerte der Kennlinie N2 wurden bei einer Viskosität von 5 cP erfasst. Die die Messwerte verbindende Kennlinie N2 wurde ebenfalls polynomisch an die Messwerte angenähert.

**Fig. 5** zeigt schematisch eine Anordnung zur Volumenstrommessung.

Wie in Fig. 5 gezeigt, ist der arterielle Leitungsabschnitt 3 an einen Lösungsbeutel 45 angeschlossen. Der Lösungsbeutel 45 kann Substituatlösung oder Dialysatlösung enthalten.

An den Lösungsbeutel 45 ist eine Waage 47 angeschlossen.

Zur Bestimmung des absoluten Drucks pumpenabwärts ist ein Drucksensor flussaufwärts der Zentrifugalpumpe 11 entbehrlich. Vorgesehen ist allein ein Drucksensor 15 flussabwärts.

Dieser Drucksensor 15 kann bei Dialysemaschinen mit Waagen für Dialysat- oder Substituatlösungsbeuteln ebenfalls eingespart werden, wenn die Höhe der Flüssigkeitssäule bis zum Pumpeneingang bekannt ist. Dieser Umstand ergibt sich daraus, dass sich der hydrostatische Druck berechnet zu

$$p(h) = \rho * g * h$$

wobei gilt:

$p(h)$ = hydrostatischer Druck als Funktion der Wasserhöhe;
$[p]$ = Pa;
g = Schwerebeschleunigung; $[g]$ = m/s$^2$;
$\rho$ = Dichte (für Wasser: $\rho$ = 1000 kg/m$^3$); $[\rho]$ = kg/m$^3$;
h = Höhe der Flüssigkeitssäule; $[h]$ = m;

Die Dichte der Lösungsflüssigkeit ist von der Lösungstemperatur abhängig. In der Regel beträgt diese 37 °C, um dem Patienten weder Wärme zu entziehen noch ihn zu erhitzen. Die Dichte kann somit als konstant und bekannt angesehen werden. In Ausnahmefällen (Überhitzung, Unterkühlung) kann dem Patienten durch eine abweichende Lösungstemperatur Wärme entzogen oder zugeführt werden. Der Betrag der sich hierbei einstellenden Differenztemperatur liegt aber in einem Bereich bevorzugt kleiner als 2 °C, besonders bevorzugt kleiner als 1 °C. Innerhalb dieser Grenzen ist die Dichte hinreichend genau als konstant anzusehen, überdies könnte die Abhängigkeit der Lösungsdichte von der Temperatur als Funktion in der Recheneinrichtung 31 der Behandlungsvorrichtung 20 oder an anderer Stelle hinterlegt sein und in die Berechnung des hydrostatischen Drucks mit einfließen.

## Patentansprüche

1. Behandlungsvorrichtung (20) zum Behandeln medizinischer Fluide, mit einer Recheneinrichtung (31), welche ausgestaltet und konfiguriert ist zum Bestimmen eines Drucks oder eines Volumenstroms eines medizinischen Fluids in einer Fluidleitung unter Einsatz wenigstens einer Zentrifugalpumpe (11), **gekennzeichnet dadurch, dass** die Zentrifugalpumpe (11) oder Abschnitte der Zentrifugalpumpe (11) Abschnitt einer Blutkassette sind; sowie dadurch, dass das Bestimmen folgenden Schritt umfasst:

   Ermitteln des Drucks stromauf der Zentrifugalpumpe (11) unter Berücksichtigung einer Angabe über einen Druck stromab der Zentrifugalpumpe (11) oder Ermitteln des Drucks stromab der Zentrifugalpumpe (11) unter Berücksichtigung einer Angabe über einen Druck stromauf der Zentrifugalpumpe (11), jeweils unter Berücksichtigen wenigstens einer Angabe über den Volumenstrom in der Fluidleitung und wenigstens einer Angabe über eine Drehzahl der Zentrifugalpumpe (11).

2. Behandlungsvorrichtung (20) nach Anspruch 1, wobei der Druck des medizinischen Fluids in einem extrakorporalen Volumenstrom bestimmt wird, welches ausgewählt ist aus Dialysierflüssigkeit, Substituatflüssigkeit, Medikamenten und Blut, insbesondere in einem arteriellen Schenkel eines extrakorporalen Blutkreislaufs, und Kombinationen hiervon.

3. Behandlungsvorrichtung (20) nach einem der vorangegangenen Ansprüche, wobei die Bestimmung des Drucks unter Berücksichtigung einer Angabe über den Volumenstrom am Eingang oder am Ausgang der Zentrifugalpumpe (11) erfolgt.

4. Behandlungsvorrichtung (20) nach einem der vorangegangenen Ansprüche, wobei die Bestimmung des Drucks unter Berücksichtigung einer Angabe über die Höhe der Fluidsäule am Eingang der Zentrifugalpumpe (11) erfolgt.

5. Behandlungsvorrichtung (20) nach einem der vorangegangenen Ansprüche, wobei die Blutkassette derart ausgestaltet ist, dass sie zur Bestimmung eines Drucks stromauf der Zentrifugalpumpe (11) nur einen Drucksensor stromab der Zentrifugalpumpe (11) aufweist.

6. Behandlungsvorrichtung (20) nach einem der vorangegangenen Ansprüche, wobei die Blutkassette derart ausgestaltet ist, dass sie zur Bestimmung eines Drucks stromab der Zentrifugalpumpe (11) nur einen Drucksensor stromauf der Zentrifugalpumpe (11) aufweist.

7. Behandlungsvorrichtung (20) nach einem der vorangegangenen Ansprüche, mit einer Einrichtung, die dazu vorgesehen und konfiguriert ist, die Zentrifugalpumpe (11) über eine magnetische Antriebsschnittstelle anzutreiben.

8. Behandlungsvorrichtung (20) nach einem der vorangegangenen Ansprüche, derart ausgestaltet, dass sie zur Bestimmung eines Drucks stromauf der Zentrifugalpumpe (11) nur einen Drucksensor stromab der Zentrifugalpumpe aufweist.

**9.** Behandlungsvorrichtung (20) nach einem der vorangegangenen Ansprüche, derart ausgestaltet, dass sie zur Bestimmung eines Drucks stromab der Zentrifugalpumpe (11) nur einen Drucksensor stromauf der Zentrifugalpumpe (11) aufweist.

**10.** Behandlungsvorrichtung (20) nach einem der vorangegangenen Ansprüche, welche als Blutbehandlungsvorrichtung, insbesondere als Hämodialysevorrichtung, ausgestaltet ist.

**Claims**

**1.** A treatment apparatus (20) for treating medical fluids with a computing device (31), which is designed and configured to determine a pressure or a volume flow of a medical fluid in a fluid line using at least one centrifugal pump (11),
**characterized in that**
the centrifugal pump (11) or sections of the centrifugal pump (11) are a section of a blood cassette; and also **in that** the determining includes the following step:

- identifying the pressure upstream of the centrifugal pump (11) taking into account an indication about a pressure downstream of the centrifugal pump (11) or identifying the pressure downstream of the centrifugal pump (11) taking into account an indication about a pressure upstream of the centrifugal pump (11), in each case taking into account at least an indication about the volume flow in the fluid line and at least an indication of a rotation speed of the centrifugal pump (11).

**2.** The treatment apparatus (20) according to claim 1, wherein the pressure of the medical fluid is determined in an extracorporeal volume flow, which is selected from dialysis liquid, substitute liquid, medicaments and blood, especially in an arterial branch of an extracorporeal blood circuit, and combinations thereof.

**3.** The treatment apparatus (20) according to anyone of the preceding claims, wherein the determination of the pressure takes place by taking into account an indication about the volume flow at the input or at the output of the centrifugal pump (11).

**4.** The treatment apparatus (20) according to anyone of the preceding claims, wherein the determination of the pressure takes place by taking into account an indication about the level of the fluid column at the input of the centrifugal pump (11).

**5.** The treatment apparatus (20) according to anyone of the preceding claims, wherein the blood cassette is designed such that it comprises only one pressure sensor downstream of the centrifugal pump (11) for determining a pressure upstream of the centrifugal pump (11).

**6.** The treatment apparatus (20) according to anyone of the preceding claims, wherein the blood cassette is designed such that it comprises only one pressure sensor upstream of the centrifugal pump (11) for determining a pressure downstream of the centrifugal pump (11).

**7.** The treatment apparatus (20) according to anyone of the preceding claims comprising a device, which is provided and configured to drive the centrifugal pump (11) via a magnetic drive interface.

**8.** The treatment apparatus (20) according to anyone of the preceding claims, which is designed such that it comprises only one pressure sensor downstream of the centrifugal pump (11) for determining a pressure upstream of the centrifugal pump (11).

**9.** The treatment apparatus (20) according to anyone of the preceding claims, which is designed such that it comprises only one pressure sensor upstream of the centrifugal pump (11) for determining a pressure downstream of the centrifugal pump (11).

**10.** The treatment apparatus (20) according to anyone of the preceding claims, which is designed as a blood treatment apparatus, in particular as a hemodialysis apparatus.

**Revendications**

**1.** Un appareil de traitement (20) destiné à traiter des fluides médicaux au moyen d'un dispositif de calcul (31) conçu et configuré pour déterminer une pression ou un débit volumétrique d'un fluide médical dans une conduite de fluide en utilisant au moins une pompe centrifuge (11),
**caractérisé en ce que**
la pompe centrifuge (11) ou des sections de la pompe centrifuge (11) représentent une section de la cassette à sang;
et aussi **en ce que** la détermination comprend l'étape suivante:

- identifier la pression en amont de la pompe centrifuge (11) en tenant compte d'une indication concernant une pression en aval de la pompe centrifuge (11) ou identifier la pression en aval de la pompe centrifuge (11) en tenant compte d'une indication concernant une pression en amont la pompe centrifuge (11), dans chaque cas

en tenant compte d'au moins une indication concernant le débit volumétrique dans la conduite de fluide et d'au moins une indication concernant la vitesse de rotation de la pompe centrifuge (11).

2. L'appareil de traitement (20) selon la première revendication, où la pression du fluide médical est déterminée dans un débit volumétrique extracorporel choisi parmi du liquide de dialyse, du liquide de substitut, des médicaments et du sang, en particulier dans une branche artérielle d'un circuit sanguin extracorporel, et des combinaisons de ceux-ci.

3. L'appareil de traitement (20) selon l'une quelconque des revendications précédentes, où la détermination de la pression a lieu en tenant compte d'une indication du débit volumétrique à l'entrée ou à la sortie de la pompe centrifuge (11).

4. L'appareil de traitement (20) selon l'une quelconque des revendications précédentes, où la détermination de la pression a lieu en tenant compte d'une indication du niveau de la colonne de fluide à l'entrée de la pompe centrifuge (11).

5. L'appareil de traitement (20) selon l'une quelconque des revendications précédentes, où la cassette à sang est conçue de telle sorte qu'elle ne comprend qu'un seul capteur de pression en aval de la pompe centrifuge (11) pour déterminer une pression en amont de la pompe centrifuge (11).

6. L'appareil de traitement (20) selon l'une quelconque des revendications précédentes, où la cassette à sang est conçue de telle sorte qu'elle ne comprend qu'un seul capteur de pression en amont de la pompe centrifuge (11) pour déterminer une pression en aval de la pompe centrifuge (11).

7. L'appareil de traitement (20) selon l'une quelconque des revendications précédentes comprenant un dispositif prévu et configuré pour entraîner la pompe centrifuge (11) via une interface d'entraînement magnétique.

8. L'appareil de traitement (20) selon l'une quelconque des revendications précédentes, conçu de telle sorte qu'il ne comprend qu'un seul capteur de pression en aval de la pompe centrifuge (11) pour déterminer une pression en amont de la pompe centrifuge (11).

9. L'appareil de traitement (20) selon l'une quelconque des revendications précédentes, conçu de telle sorte qu'il ne comprend qu'un seul capteur de pression en amont de la pompe centrifuge (11) pour déterminer une pression en aval de la pompe centrifuge (11).

10. L'appareil de traitement (20) selon l'une quelconque des revendications précédentes, conçu comme un appareil de traitement du sang, en particulier comme un appareil d'hémodialyse.

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19840399 A1
- US 6654627 B1
- WO 2005085772 A1
- EP 1284415 B1

**In der Beschreibung aufgeführte Nicht-Patentliteratur**

- Bilanziereinrichtung, externe medizinische Funktionseinrichtung, Behandlungsvorrichtung sowie Verfahren. *eingereichten Anmeldung 10 2009 058 681.4,* 16. Dezember 2009